# EUROPEAN PATENT SPECIFICATION

(11) **EP 1 446 891 B1**
(45) Date of publication and mention of the grant of the patent: **21.08.2013**
(21) Application number: 02789277.7
(22) Date of filing: 24.10.2002
(51) Int. Cl.: H04B 1/38, G08B 21/06, B60R 11/02

(54) **SAFETY CONTROL SYSTEM FOR VEHICLES**
SICHERHEITSSTEUERSYSTEM FÜR FAHRZEUGE
SYSTEME DE CONTROLE DE SECURITE POUR VEHICULES

(30) Priority: 24.10.2001 US 336293 P; 21.06.2002 US 390877 P
(43) Date of publication of application: 18.08.2004
(73) Proprietor: Naboulsi, Mouhamad Ahmad, West Bloomfield, MI 48323 (US)
(72) Inventor: Naboulsi, Mouhamad Ahmad, West Bloomfield, MI 48323 (US)
(74) Representative: Ström & Gulliksson AB
(86) International application number: PCT/US2002/034170
(87) International publication number: WO 2003/036805

(56) References cited:
- WO-A-98/05543
- US-A- 5 266 922
- US-B1- 6 188 315
- US-B1- 6 256 558
- US-B1- 6 353 778

## Description

### Field of the Invention

The present invention relates to the field of telematics, namely to the field of integrating information, communication, computing and entertainment technologies into vehicles for civilian or military use. The invention particularly relates to safety control systems for vehicles for avoiding potentially dangerous conditions tending to produce accidents.

### Background of the Invention

One potentially dangerous condition is the use of a vehicle telephone by the vehicle driver while driving the vehicle. The use of telematics in general and particularly cellular telephones by drivers while driving has been found to increase the possibility of an accident since such a telephone not only diverts the driver's attention from driving, but also generally requires the use of at least one of the driver's hands and distract the driver's eyes from the road and traffic. In fact, many states and countries have enacted legislation requiring that telephones used in vehicles by drivers while driving must be of the "hands free" type and usually telematics equipment carries a warning to educate and discourage the driver about the risk of using while driving. However, such legislation is difficult to enforce and education is not usually effective in assuring driver compliance. Moreover, even where the vehicle is equipped with a "hands free" telephone, drivers nevertheless still frequently use one hand for holding or dialing the telephone. When one hand is occupied by holding a telephone, the danger of causing an accident in an emergency situation is increased because of the additional reaction time required to properly grip the steering wheel with both hands.

There are other potentially dangerous conditions and inherent risks in driving that depends on the driving act itself, such as accelerating, decelerating, excessive maneuvering, merging to or exiting a freeway, passing, changing lanes, changing gears, depressing the clutch, high speed, negotiating a turn, braking, reverse-driving, or a stress condition on the part of the driver, which could increase the possibility of an accident should the driver be distracted by the telephone. This inherent risk is also dependent on the driving purpose as well, for example, the risk in driving a police cruiser is inherently riskier then in driving a sedan, and driving a delivery van has different risk than driving the family van.

Herbert et al. , U. S. Patent 6,188, 315 and Brown, U. S. Patent 6,353, 778, disclose systems for avoiding preset potentially dangerous conditions while operating a vehicle having a vehicle telephone, but the systems described in those patents are of relatively limited application, and do not provide for avoiding dangerous conditions or to managing risk and individualizing the warnings to individual driving skills or application and to combinations of events and environmental conditions.

US 6,256,558 B1 describes a vehicle display system with drive enforcement control. This system keeps the driver from inputting data into the system while driving a vehicle, but it permits a person in the front passenger seat to perform the data input operations. When the drivers grips the steering wheel and thereby depresses drive enforcement releasing switches located on the vehicle steering wheel, the main ECU releases the drive enforcement state, thereby enabling passengers other than the driver to perform data input operations.

### Objects and Brief Summary of the Invention

The object of the present invention is to provide a safety control system for vehicles tending to reduce the possibility of accidents in one or more of the above respects.

According to one aspect of the present invention, there is provided a safety control system for vehicles including a telephone and sensor means for sensing a potentially dangerous condition and for automatically disabling the telephone when sensing such condition; characterized in that the sensor means includes two sensors mounted on a steering member to be gripped by the two hands of the driver of the vehicle and effective to suspend use of the telephone when the two hands of the driver are not sensed as gripping the steering wheel while the vehicle is in motion. This system is modular, dynamic, interactive, and adaptive to each individualized user. The invention employs a method for automated machine prioritizing to provide assistance the to driver and to optimize the functionality of telematics features accessibility by arranging them according to a user's needs and preferences in the operation based on usage frequency of individual features and/or application or as customized individually by the user preferences, skills and events.

According to further features in the described preferred embodiment, the sensor means includes two sensors on opposite sides of the steering wheel located to sense the proper gripping of the steering wheel by the two hands of the driver; preferably, the two sensors being located approximately on or between the "two" and "ten" and the "three" and "nine" clock positions of the steering wheel.

It will thus be seen that such a system, requiring both hands to be on the steering wheel in order for the driver to operate the telephone/telematics, not only requires the vehicle to be equipped with a "hands free" telephone/telematics system, or a telephone/telematics system that can be used as such with an adapter or when docked to the system gateway, but also enforces the use of the "hands free" feature by sensing that the driver actually has both hands placed on the steering member before the telephone or other telematics can be operated. Disabling the operation of the telephone would preferably include not only disabling making outgoing and receiving incoming telephone calls, but also disabling the ringing signal of an incoming call since such a ringing signal could be particularly distracting to the driver in a critical situation.

According to further features in the described preferred embodiment, the vehicle may also include a computer or the driver may also use a portable multi-function telematics device in the vehicle allowing access to the Internet for transmitting and/or receiving faxes or e-mail or browsing the web or accessing a WAN, the sensor means also disabling driver initiated access when the two hands of the driver are not sensed as gripping the steering member while the vehicle is in motion.

According to further optional features in the preferred embodiment of the invention described below, the sensor means may further include means for sensing accelerating, decelerating, merging to or exiting a freeway, passing, changing lanes, changing gears, depressing the clutch a reverse-drive condition of the vehicle, the braking of the vehicle, the undue proximity of the vehicle to another vehicle, excessive maneuvering, and/or an unduly high velocity of the vehicle, any one of which conditions, or combination of conditions, may also be effective to disable the operation of the telephone, computer, or other potentially distracting equipment within the vehicle.

According to still further optional features in the preferred embodiment of the invention described below, at least one of the sensors on the steering member also senses a physiological condition of the driver and disables the telephone when a predetermined physiological condition is sensed. For example, the physiological conditions sensed could be a predetermined gripping force applied by a hand of the driver while gripping the steering wheel, or a predetermined pulse rate, temperature, blood pressure, and/or skin conductivity of the driver. Such physiological condition may indicate a stress condition of the driver and, when sensed, disable the incoming operation of the telephone so as not to aggravate the stressed condition.

The system may also include means for indicating a drowsiness condition. For example, the system may include a steering direction sensor which actuates a drowsiness alarm when sensing a failure to change the steering direction within a predetermined time, distance interval while accounting for vehicle speed in indicating a possible drowsiness condition in the driver. Additionally, such sensor when monitored with respect to changes over time will indicate jerk reaction, which indicates that the driver was not paying attention and the system will temporarily suspend all telematics to give the driver a chance to recover. Another application for such a sensor is the monitoring of an OFF Zero angle for an extended period of time/distance which can indicate a blind curve or hard curve, and again, here the system will temporarily suspend telematics of all functions from interacting with the driver and vice versa until normal driving functions are restored.

According to further features in the described preferred embodiment, the telephone may also be disabled when the vehicle is traveling in the reverse direction, or is being braked, or is within a predetermined proximity of another vehicle, or is traveling at a high velocity accelerating, decelerating, merging to or exiting a freeway, passing, changing lanes, changing gears, depressing the clutch, or a driver is occupied using other accessories in the vehicle. Since a high degree of attention of the driver is required under all the foregoing conditions, operation of the vehicle telephone, even the ringing signal of an incoming telephone call, could be highly distracting to the driver and is therefore disabled to avoid the possibility of increasing the risk of an accident.

Further features and advantages of the invention will be apparent from the description below.

### Brief Description of the Drawings

The invention is herein described, by way of example only, with reference to the accompanying drawings, wherein:
Fig. 1 schematically illustrates one form of safety control system for vehicles constructed in accordance with the present invention;
Fig. 2 is an enlarged view illustrating the steering wheel in the vehicle of Fig. 1 and the sensors mounted thereon;
Fig. 3 is a block diagram illustrating the main components in the system of Fig. 1;
Fig. 4 is a flowchart illustrating the operation of the system of Fig. 1; and

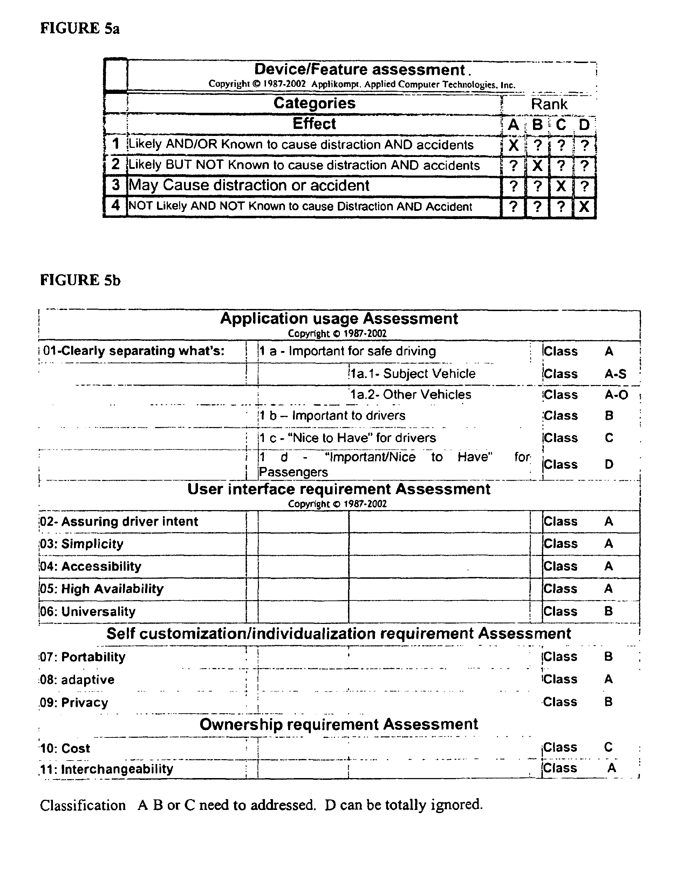

Figs. 5a and 5b shows an assessment process that may be used to customize a system for a specific application and to asses the safety of any of the telematics devices or functions.

### Description of Preferred Embodiment

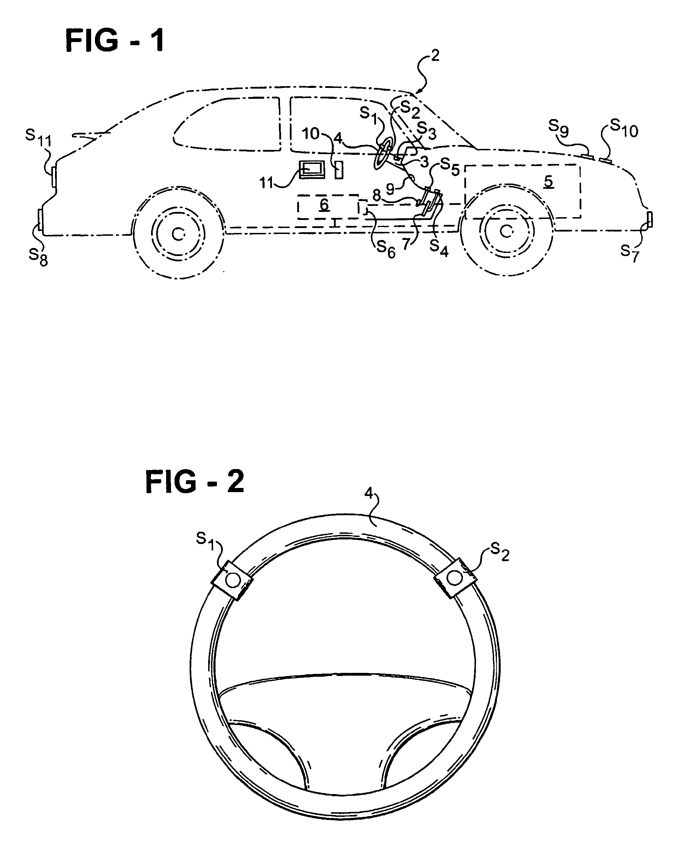

Fig. 1 schematically illustrates a vehicle, generally designated 2, equipped with a control system for sensing a variety of risk factors and potentially dangerous conditions and for automatically executing various responses when sensing such conditions in order to avoid hazardous situations tending to increase the possibility of an accident. A particularly hazardous situation avoided by the control system illustrated in Fig. 1 is the use of the vehicle telephone or other telematics such as e-mail, incoming page or the like in certain situations wherein a making of a telephone call by the vehicle driver, or the receiving of an incoming call, particularly the ringing current of such a call, may be so distracting to the driver as to increase the possibility of an accident in the event the driver is in a high-risk driving situation. In such cases, the vehicle telephone and other telematics are suppressed and no incoming telematics are allowed to distract the driver. In case the driver is the party initiating the telematics, a visual indicator and audio feedback is activated to indicate to the driver that telematics is disabled and supply reason and recommend driving modification to enable telematics. Another condition sensed by the system is undue stress in the driver, as indicated by the sensed pulse rate, temperature, blood pressure, skin conductivity (e.g. perspiration), loud voice(s) or stressful sounds in the cabin, such as baby crying, dog barking etc., any combination of one or more of which conditions would also disable incoming telematics. A further condition sensed by the system is the possibility of drowsiness on the part of the driver, in which case an audio alarm would be activated to alert the driver to this condition. Other alarms to overcome driver drowsiness would include vibration in the seat, changing HVAC temperature settings and blower speed to extremes, etc. The system will restore telematics when conditions are normalized and will notify driver of all missed activities.

Vehicle 2 illustrated in Fig. 1 is a conventional vehicle including a steering mechanism, generally designated 3, having a steering wheel 4, a propulsion device such as a motor or engine 5 for driving the vehicle via a transmission or other torque converting means schematically indicated 6, an acceleration pedal 7, and a braking pedal 8 for controlling the vehicle. Vehicle 2 further includes one or more visual indicator and audio alarms 9, e.g. mounted within the forward-look ahead viewing or hearing by the driver.

Fig. 1 further schematically illustrates a cellular telephone 10 within the vehicle, and a computer 11 or other multifunction telematic device allowing access to the Internet for transmitting and/or receiving faxes or e-mail, WAN and Web access. Vehicle 2 illustrated in Fig. 1 may also include many other components conventionally provided on vehicles at the present time or to be provided in the future.

The safety control system included in vehicle 2 illustrated in Fig. 1 includes a plurality of sensors for sensing various conditions with respect to the vehicle driver and/or the vehicle itself. These signals are collected via direct taping to existing or added sensors or via vehicle bus and user specified values. These include sensors S₁ and S₂ applied to the steering wheel 4 of the vehicle; sensor S₃ applied to the steering mechanism 3 of the vehicle to sense changes in the steering direction; sensor S₄ sensing the condition of the gas pedal 7; sensor S₅ sensing the condition of the braking pedal 8; and sensor S₆ sensing the condition of the transmission or other type torque converter 6.

Also schematically illustrated in Fig. 1 are sensors S₇ and S₈ carried to sense the proximity of the vehicle with respect to another vehicle; sensor S₉ sensing darkness or alternatively sensing the activation of the headlight; and sensor S₁₀ sensing rain or alternatively sensing the activation of the front or rear wipers or headlight wipers.

As will be described more particularly below, the foregoing sensors (or signals) are generally effective only when the vehicle is moving to sense their respective conditions and to execute certain control functions in order to decrease the possibility of an accident. One important control function is to disable an incoming call from ringing the telephone 10, and the computer or other telematics portable or built in 11 from accessing the Internet or announcing incoming signals, e.g. page, e-mail etc., and to indicate same by actuating a visual indicator and an audio feedback if a driver attempts to initiate telematics during an unsafe or a high risk condition 9 and may direct a driver to alternative driving habit to gain access to telematics. The system will restore telematics when conditions are normalized and will notify driver of all missed activities. In some cases, such as where a drowsiness condition is sensed, an audio alarm 9 is actuated. Other alarms to overcome driver drowsiness would include vibration in the seat, changing HVAC temperature settings and blower speed to extremes, etc.

Fig. 2 more particularly illustrates the sensors S₁, S₂ mounted on the steering wheel 4. As shown in Fig. 2, the two sensors are mounted on or between the "two" and "ten" and the "three" and "nine" clock positions of the steering wheel 4; the "two" and "ten" positions are considered to be the most preferred ones for the two hands of the driver in order to manipulate the steering wheel, but other positions could be employed, such as "nine and fifteen", which provide more clearance for activated airbags. The two sensors S₁, S₂ thus sense the proper positioning of the two hands of the driver on the steering wheel 4.

The two sensors S₁, S₂, which may be attached to or embedded in the steering wheel, may be simple electrical switches which are actuated by the respective hand of the driver when properly gripping the steering wheel. Preferably, however, one or both of the sensors S₁, S₂ or other sensors are also capable of sensing a physiological condition of the driver, such as the gripping force applied by the driver's hand, or the pulse rate, blood pressure, temperature and/or electrical skin conductivity of the driver's hand while gripping the steering wheel. For example, sensor S₁ could include a transducer for converting pressure to an electrical signal, such as a spring-type, carbon-type transducer, optical type or semiconductor type. Sensor S₂ could include one or more transducers, such as known in finger probes, for sensing pulse rate, temperature, and/or electrical skin conductivity, and for outputting an electrical signal corresponding to the magnitude of the sensed condition, as described for example in U.S. Patents 6,319,205; 5,438,986; 5,065,749; 4,860,759; 6,415,176 or 5,897,505.

As will be described more particularly below, sensors S₁ and S₂ thus sense that both the driver's hands properly grip both sides of the steering wheel 4 to enable operation of the telephone 10 and the computer 11 or similar multi-function or standalone telematics devices. Thus, the telephone 10 can be permitting "hands free operation" or a telephone/telematics system that can be used as such with an adapter or when docked to the system gateway, as required by many laws to avoid accidents, but also the driver is permitted to use the telephone only in a "hands free" manner, thereby precluding the driver from gripping a telephone to operate it even though the telephone or the telematics system may has a "hands free" capability.

In addition, by providing sensor S₁ and/or sensor S₂ with the capability of sensing a physiological condition of the driver while gripping the steering wheel, other conditions can be sensed to disable the telephone for further reducing the possibility of an accident. For example, the gripping force applied by one or both hands of the driver may indicate a stress condition of the driver. A stressed condition may be also indicated by the sensed pulse rate, temperature and/or electrical skin conductivity (the latter indicating perspiration) of the driver. If a stress condition is sensed, the telephone 10 is disabled so as to decrease the possibility that the ringing noise of an incoming telephone call will so distract the stressed driver as to create a hazardous condition, or that the making of an outgoing call by the driver will be so distracting to the stressed driver as to create a hazardous condition.

The provision of a grip sensor on the steering wheel also enables the system to sense drowsiness or dozing of the driver, as in U.S. Patent 4,485,375, incorporated herein by reference. Thus, if the gripping force sensed by sensor S₁ and/or sensor S₂ drops while the vehicle is in motion, this could indicate a drowsiness condition. If such a condition is sensed, the audio alarm 9, or alternatively a vibrator, may be activated, together with a visual indicator 8, in an attempt to arouse the driver and to alert the driver to the drowsiness condition. When drowsiness is sensed, the telephone 10 would not be disabled since the ringing of an incoming call may be further effective to arouse the driver. Other alarms to overcome driver drowsiness would include vibration in the seat, changing HVAC temperature settings and/or blower speed to extremes, etc.

The sensors S₁ and S₂ are preferably located at the ten o'clock and two o'clock positions but may be alternatively located in other positions such as the nine o'clock and three o'clock positions. The mechanisms of the switch include a jog switch and slide switch and a rocker switch. The sensors can be arranged to be actuated either in the thumbs-up position or the thumbs-down position. The sensors are tested for integrity by the microprocessor 20 during start up and are designed so as not to be triggered by accidents. The detection of failed switches will cause the microprocessor to block operation of the system.

Sensor S₃ is coupled to the steering mechanism 3 so as to sense changes in the steering direction. For example, an alert driver constantly makes minor changes in the steering direction automatically, but not so with respect to a drowsy or dozing driver. Accordingly, if sensor S₃ fails to sense a change in the steering direction within a predetermined time interval, this would indicate a possible drowsiness condition in the driver, and therefore the audio alarm 9 would be activated in an attempt to arouse the driver and alert him to that condition. Other alarms to overcome driver drowsiness would include vibration in the seat, changing HVAC temperature settings and blower speed to extremes, etc. or changing recline status or CD tracks and volumes to extremes.

Sensor S₄ senses the depression of the gas pedal 7, sensor S₅ senses the depression of the brake pedal 8, and sensor S₆ senses the condition of the transmission 6 and/or also the velocity of the vehicle. For example, if the transmission is in reverse gear, the driver should not be distracted by receiving or making a telephone call, and therefore the telephone should be disabled. If desired, the same could apply in any gear other than the normal drive gear. Also, if the vehicle is moving at a relatively high velocity, or is engaged in turning or otherwise rapidly maneuvering, such that any unnecessary distraction of the driver should be avoided, the telephone could likewise be disabled.

Sensor S₇ mounted at the front of the vehicle senses its proximity to a vehicle ahead of it; sensor S₈ mounted at the rear of the vehicle senses the proximity of a vehicle behind it; sensor S₉ senses the darkness level of the road on which the vehicle is traveling (e.g., whether day or night, whether the road is brightly illuminated); sensor S₁₀ senses a rain condition; and sensor S₁₁ senses whether either of the turn indicators of the vehicle is operating to signal for a turn or a change of lanes.

The conditions sensed by sensors 8₇-S₁₁ are also such that a hazard may be produced if, during the existence of such a condition, the full attention of the driver would be diverted by the ringing of the telephone or by the use of the telephone for making an outgoing call. Accordingly, under such conditions, the telephone 10 is disabled from operation. Similarly, the computer 11, if present, is disabled from operation to preclude access to the Internet for transmitting and/or receiving faxes or e-mail, which could also result in a similar distraction increasing the possibility of causing an accident.

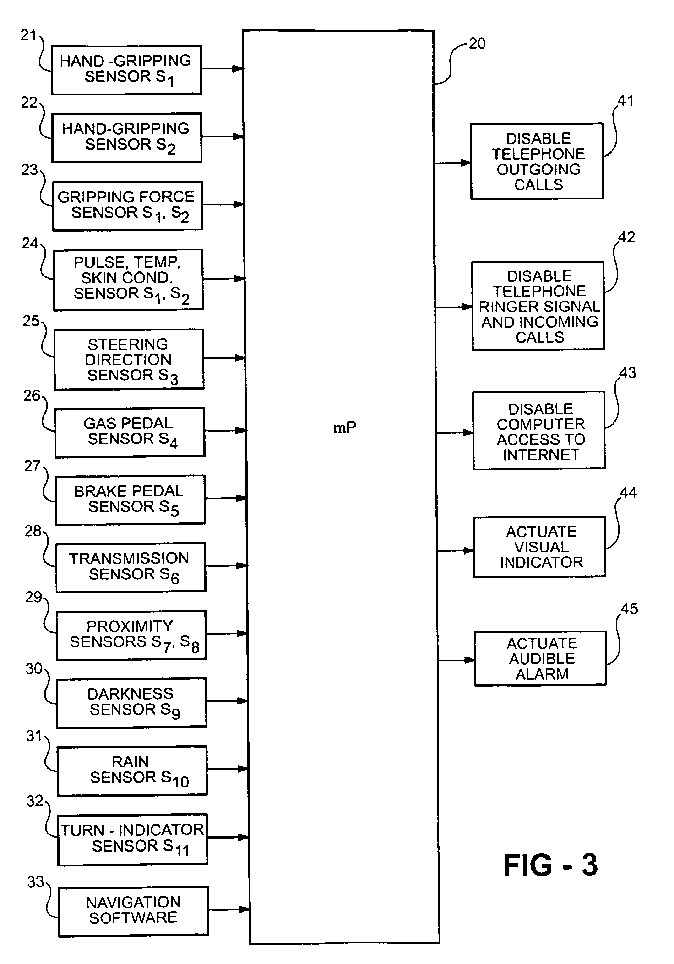

Fig. 3 is a block diagram schematically illustrating a microprocessor, generally designated 20, included in the vehicle safety control system of Fig. 1, together with its inputs schematically indicated by block 21-30, and the outputs schematically indicated by blocks 31-35.

Thus, as shown in Fig. 3, microprocessor 20 includes inputs 21 and 22 from the steering wheel sensors S₁, S₂, to indicate whether the steering wheel is being properly gripped by the two hands of the driver. Microprocessor 20 further includes an input 23 indicating the gripping force applied by one or both of the hands to the sensors S₁, S₂, and an input 24, also from one or both of the sensors S₁, S₂, indicating the pulse, skin conductivity, temperature and/or other physiological condition of the driver having a bearing on proneness of the driver to accidents. As indicated earlier, these inputs indicate particularly whether the driver is in a stressed condition, drowsy, or in an alternate embodiment, when an optional breath alcohol sensor is activated.

Another input into microprocessor 20 is from the steering direction sensor S₃, as indicated by block 25. This input is helpful in indicating the alertness of the driver, particularly whether the driver may be in a drowsy or even a dozing state, which would be indicated if this input shows no change in the steering direction within a predetermined period of time.

Another input to the microprocessor would be from a sensor associated with the vehicle cup holder to indicate when a cup which was initially disposed in the holder has been removed, as for drinking. The sensor might include a weight indicator to determine whether the cup was empty when lifted or a temperature sensor to sense heated beverages.

Further inputs into microprocessor 20 include signals from the gas pedal sensor S₄ to indicate high acceleration (block 26); the braking pedal sensor S₅ to indicate braking (block 27); the transmission sensor S₆ to indicate high vehicle speed or reverse drive (block 28); the proximity sensors S₇, S₈ at the opposite ends of the vehicle to indicate the proximity of the vehicle to other vehicles (block 29); the darkness sensor S₉ (block 30); the rain sensor S₁₀ (block 31); and turn-indicator sensors S₁₁ (block 32), and other sensors such as vehicle speed.

Fig. 3 illustrates a further input from navigation software (block 33) with which the vehicle may be equipped in order to assist the driver in navigating the vehicle to various desired locations. For example, the navigation software could be pre-programmed to output a signal to microprocessor 20 at certain locations, such as at heavily-trafficked roads, intersections, bridges, tunnels, etc., where the full concentration of the driver is sufficiently critical to avoid distractions as may be caused by a telephone call.

It will be appreciated that other sensors could be provided as inputs into microprocessor 20 wherein similar conditions may occur, either on the part of the driver, the vehicle, and/or the environment, in which, for purposes of safety, external distractions are to be avoided such as may be caused by making or receiving a telephone call.

In the preferred embodiment of the invention, the microprocessor 20, among other functions, acts as a "state machine" to define, arrange and prioritize features and functionalities of the system. In other applications this function can be performed by standalone which interconnects with a microprocessor 20. The state machine aspect of the microprocessor may make telematic control decisions on a variety of criteria such as: (a) the frequency of use of the application, the frequency in which a number, e-mail or URL is contacted; (b) based on safety/urgency priorities, e.g. cruise or CD changer, cell messages or other telematics, or music played on the radio; (c) as preset by the operator; (d) optionally, based on other collected information from the driving system, the microprocessor will initiate calls at predetermined times out of voice mail as, for example, when the driver completes backing out of a driveway and begins a trip.

The user provides signals to the state machine to block features or incoming telematics based on ID, location of phone numbers, e-mail addresses or URL. The blocked or stored telematics will be announced to the driver or stored for use in controlling the system in the future.

The state machine employs an assessment of the incoming cells and places them in categories such as: (a) likely and/or known to cause distraction and accidents; (b) likely but not known to cause distraction and accidents; (c) may cause distraction or accidents; (d) not likely and not known to cause distraction and accidents. These categories will be used to determine the effect of the incoming signals on the telematic system in accordance with the following Table 1:

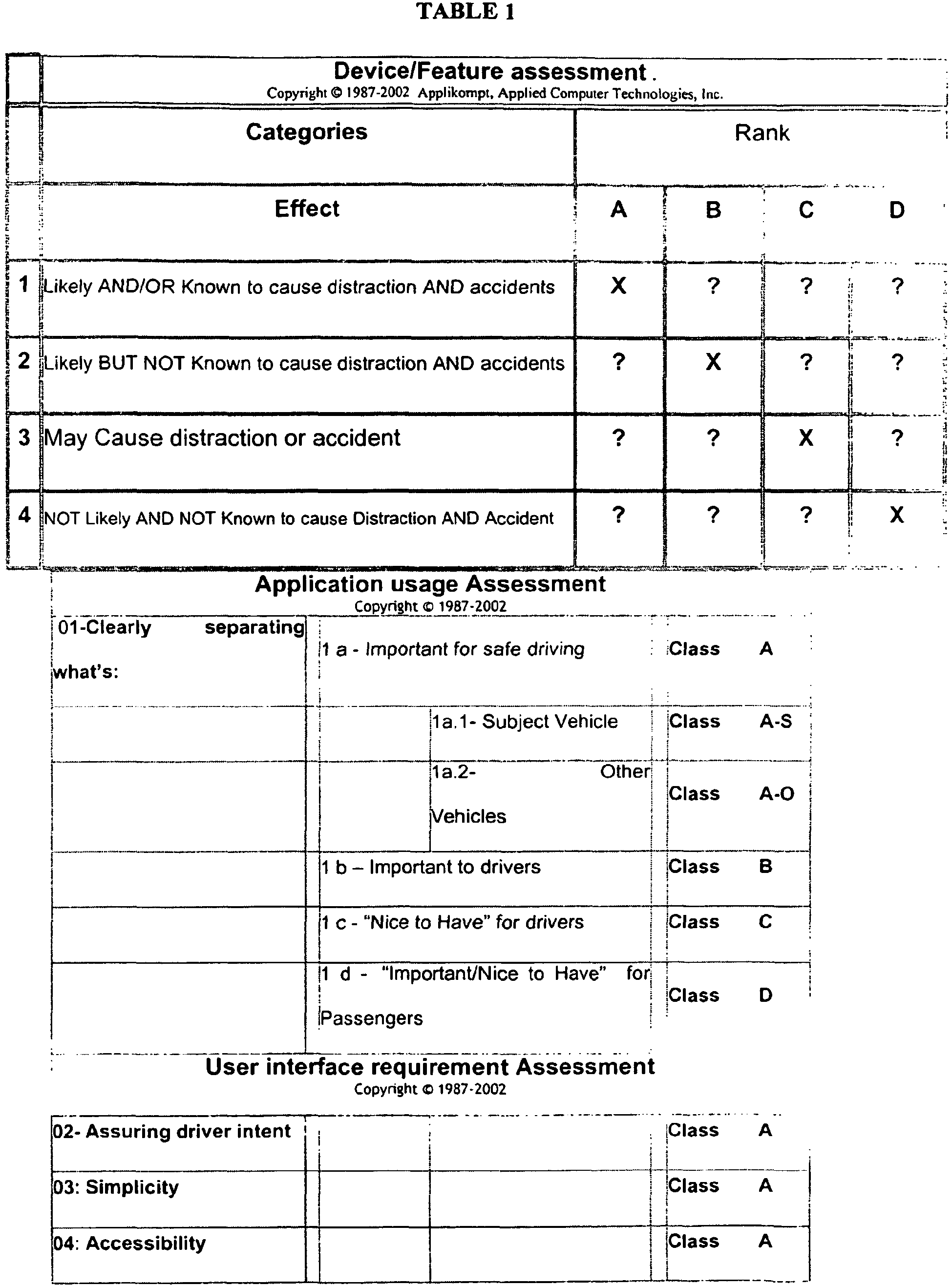

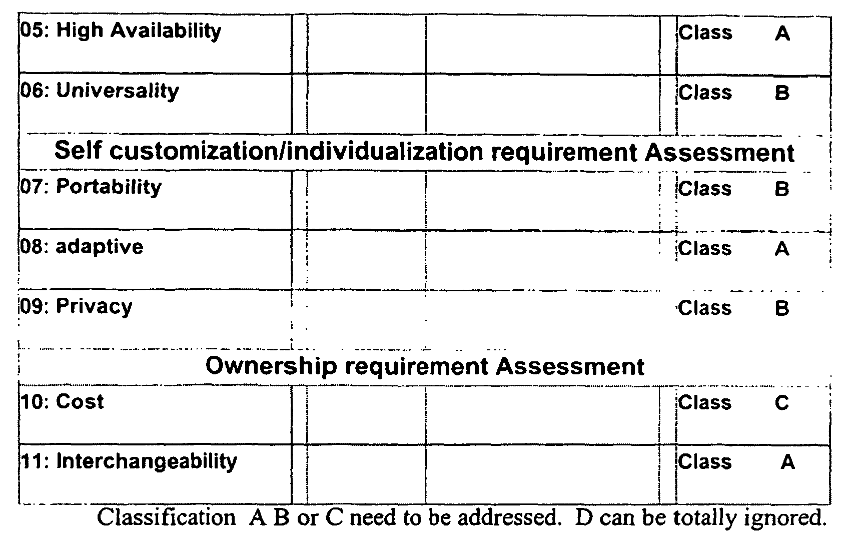

The outputs from microprocessor 20 include control signals as shown by the following blocks: block 41, effective to disable the telephone or other telematics from making outgoing calls; block 42, effective to disable the telephone from receiving incoming calls and from actuating the ringing signal; block 43, effective to disable the computer, if provided, from accessing the Internet to make or receive e-mail, faxes, etc.; block 44, effective to actuate a visual indicator viewable by the driver; and block 45, effective to actuate an audible alarm.

### Operation

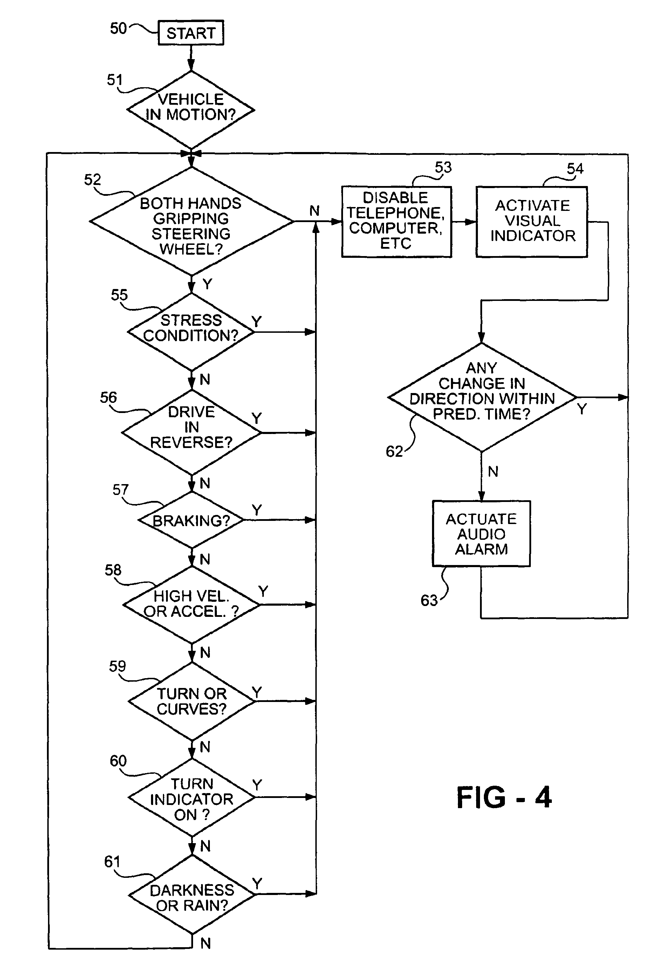

Fig. 4 is a flowchart illustrating an example of the operation of the system of Figs. 1-3.

Thus, as shown in Fig. 4, the control system is made operational when the vehicle is in motion (blocks 50, 51). When the vehicle is in motion, a microprocessor 20 outputs signals 41, 42 and 43 (Fig. 3) disabling the vehicle telephone, computer, etc. within the vehicle (block 53), and also signal 44 actuating a visual indicator within the vehicle to indicate this condition (block 54).

If, on the other hand, both hands of the driver are properly gripping the steering wheel 4 so as to actuate the two sensors S₁, S₂, one or both of the sensors is used to sense a physiological condition of the driver that might indicate a stress condition (block 55). For example, such a stress condition could be indicated by an unduly high gripping force applied by one or both of the hands of the driver to the steering wheel, or by an unduly high pulse rate of the driver or skin conductivity of the driver indicating a high degree of perspiration. If such a stress condition is indicated as being present, the telephone, computer, etc. are also disabled (block 53), and a visual indicator activated (block 54) to indicate this condition.

Next, the system checks to determine the condition of the vehicle, e.g. whether the vehicle: is traveling in reverse, as indicated by sensor S₆ (block 56); is being braked, as indicated by sensor S₅ (block 57); is traveling at or over a predetermined high velocity or high acceleration, as indicated by sensor S₆ (block 58); is executing a curve or turn, as indicated by steering mechanism sensor S₃ (block 59); is about to execute a turn, as indicated by turn indicator sensor S₁₁ (block 60); or is traveling in the dark or in the rain, as indicated by sensor S₉ or sensor S₁₀ (block 61). If any of these conditions is sensed, the telephone and the Internet access by the computer are also disabled (block 53), and a visual indicator is actuated to indicate this condition (block 54).

As further shown in Fig. 4, if while the vehicle is in motion no change in steering direction has been sensed within a predetermined time interval (block 62), an audio alarm or vibrator is also activated (block 63) to alert the driver to a possible drowsiness or dozing condition. Other alarms to overcome driver drowsiness would include vibration in the seat, changing HVAC temperature settings and/or blower speed to extremes, etc.

If desired, a manual override switch can be provided to enable the driver to manually override any of these controls, preferably except for the control of block 52 assuring that both hands of the driver are properly gripping the steering wheel.

### Setup Scenario:

Driver set up a portable Telematic device such as a cell phone, or a web page etc. With driver preferences:
(1) Control preferences ,e.g. Hands always Vs Hands on for Telematics only
(2) Annoyance items: Baby crying, Dog barking, smokers in car etc.
(3) Telematics option: Preferred application to use, preferred priority system etc.
(4) Other emergency and identifying information.
(5) A driver enters a vehicle
   a. docks all electronic communication equipment, e.g. pager, cell phone, PDA, etc., to the control system wirelessly or physically, thus identifies him/herself to the vehicle
   b. System mutes all Telematics but keeps them active
   c. Driver initiates his/her trip.

### Scenario One (Driver Initiated)

The driver wants to make a call, review pages, read e-mail or connect to the Internet. (1) The driver will activate the safety switch and then, after the system acknowledges safety switch activation by providing the driver with a beep or voice feedback, the driver with his/her hand on the actuated safety switch will toggle through options with the toggle switch until he gets to a selection that is needed, then using the toggle switch will confirm selection and proceed with the desired action. This could be multiple layers of options. These options can be provided on a HUD or via voice. Even if devices can be activated by voice control, they still need to have the safety switch depressed to ensure driver intention and not an erroneous sound from the radio or a passenger or a malfunction of devices. During this time the driver's hands must remain at 10/10. The driver must maintain the steering wheel within a specific angle which is calculated based on the following inputs: (1) weather condition, (2) speed of vehicle, (3) proximity of vehicle to others (front/back), feedback from ABS, ESP, traction control, etc. This angle (for example) is about 30 degrees either side of zero if the speed is 40 mph, but it is less when the speed is higher and more when the speed is lower. The driver will also be allowed to temporarily take his hands off the 10/10 position to, for example, make a sharp turn but will have to put them back at 10/10 to continue the previous activity. This amount of time is again dependent on speed, weather, vehicle proximity to others and feedback from ABS, ESP and traction control.

### Scenario Two (Incoming)

Incoming information will be customized by the driver, in accordance with Table A, to select what he/she wants to receive and in what priority. Once incoming information is detected by the system, the system will go through a checklist to verify feedback from steering about position and about speed and ABS and ESP and traction control and weather condition. When all conditions are met, the system will announce the incoming information to the driver who will have to press the safety control switch and hold up. While using the toggle switch to accept the incoming information, the remainder of the controls will be as per outgoing, including hands at 10/10 and hands off for a certain temporary amount of time.

It will thus be seen that the illustrated system is effective to disable the operation of the telephone (and/or access to the Internet by a computer) within the vehicle when any of the above-described conditions is sensed, to thereby avoid a distraction which may cause accidents. The fact that both hands of the driver must be gripping the steering wheel in order to enable the operation of the telephone (and/or computer) not only requires that the vehicle must be equipped with a "hands free" capability, but that the driver must actually use this "hands free" capability created by the system gateway in order to make or receive telephone calls or other telematics activities. In addition, other sensors could also be provided to disable a vehicle telephone or a multi-function telematics system or Internet access provided by a vehicle computer in response to other conditions, such as the detection within the vehicle of the sounds of an emergency siren in an approaching vehicle, a child crying within the vehicle, the driver use of a drink from a monitored cup holder or a monitored food tray, or the activities such as modifying the cabin temperature, changing the volume on the radio, extending the sun visor etc.

The monitoring of all such signals, sensors, data and conditions is done by a modular dynamic plug and play state machine that integrates, prioritizes, enables, blocks or mutes telematics application and telematics functionalities based on priorities determined by learning frequency and characteristics of use or by driver preset preferences.

Such machine may be a hardware based, a software embedded in a dedicated hardware or a software/protocol embedded in one or more telematic equipment and it may act as a node on a network of telematic equipment and the vehicle bus, or as a hub for all telematics and a gateway to the vehicle, or any combination of the above.

The state machine can allow driver to set their preferences on a portable telematics device such as a cellular phone, or a WAN, Web site or via a FTP and e-mail. Such set up can be transferred to the vehicle in use when the driver docks the cell phone or other portable telematics devices to the system gateway. The downloaded profile will be updated with driving skills, driver habits and geographical/time/date based notes added by the driver while driving. The updated profile will be uploaded back to the source when the vehicle comes to a final stop, or ongoing as driving is being carried out. Such data may be direct values and status or a statistical representation of a driving experience.

The preferences included by the driver will range from telematics management options, e.g. preset priorities or automatic based on learning by frequency of use, tags of time, location and physiology. Preset priorities will allow a driver to assign sequence of access to telematics and telematics functionalities or to block certain activities based on time of day or source of telematics or geography at will. Automatic based learning condition, on the other hand, for example, if the driver physiology shows stress during a telephone conversation with a certain number, such number will be tagged and will be treated as a source of high risk and will be blocked during unusually risky conditions so a driver does not engage in additional cognitive hungry activities. Additionally, if a driver uses telematics device A more often the B which is used less often then C, the access to such devices will be based on the mostly used first. In this case, A is followed by C and C is followed by B. Similar frequency based access priorities are applied to function of such telematics and also prioritized based on time, geography etc.

Other preferences set by the driver can include emergency contacts, medical record summary or identification, etc. to be used along with telemetry data when automatically reporting an accident via text to speech and via e-mail. This will help emergency dispatch understand and prepare the correct type of help needed, e.g. number of passengers, fire in cabin, impact speed, driver physiology and the driving telemetry before and during the impact.

The decisions to block, enable etc are accomplished by algorithms that share the hosts of signals provided to monitor for specific conditions that portrays. These algorithms also update the driver profile to include skills and habits for further relaxing or restricting telematics. For example, a driver that drives frequently on expressways and in close proximity to other vehicles will be allowed more leeway then a person that hardly drives on the expressway. Similar monitoring occurs for nighttime driving, adverse weather driving and so on.

The invention is defined by the claims. While the invention has been described with respect to one preferred embodiment, many other variations, modifications and applications of the invention may be made, falling under the scope of the claims.

## Claims

1. A safety control system for vehicles (2) requiring both hands to be on the steering wheel in order for the driver to operate a telephone or telematics, said system including a telematics device (10, 11) and sensor means (S₁-S₁₁) for sensing a potentially dangerous condition and for automatically disabling use of the telematics device (10, 11) when sensing such condition;
**characterized in that** said sensor means (S₁-S₁₁) includes
two sensors (S₁, S₂) mounted on a steering wheel (4) to be gripped by the two hands of the driver of the vehicle (2) and effective to suspend or disable the telematics device (10, 11) when the two hands of the driver are not sensed as gripping said steering wheel (4) while the vehicle (2) is in motion, wherein
the two sensors (S₁, S₂) are electrical switches which are arranged to be actuated either in the thumbs-up position or the thumbs-down position by the respective hand of the driver when properly gripping the steering wheel (4), whereby
the driver with his/her hand on the actuated safety switch is allowed to toggle through options with the safety switch until he gets to a selection that is needed.

2. The system according to claim 1, wherein said system is capable of disabling the ringing signal of an incoming call.

3. The system according to claim 1, wherein said telematics device (10, 11) is a telephone.

4. The system according to claim 1, wherein said two sensors (S₁, S₂) are located approximately on or between the "two" and "ten" and the "three" and "nine" clock positions of the steering wheel (4).

5. The system according to claim 1, wherein said vehicle further comprises a computer or multifunction telematics device allowing access to the Internet for transmitting and/or receiving faxes or e-mail, WAN or browsing websites, and wherein said sensor means (S₁, S₁₁) disables said access when the two hands of the driver are not sensed as gripping the steering wheel (4) while the vehicle is in motion and the risky dangerous conditions are taking place.

6. The system according to claim 1, wherein the sensor means (S₁-S₁₁) include means for sensing accelerating, decelerating, merging to or exiting a freeway, passing, changing lanes, changing gears, depressing the clutch, a reverse drive condition of the vehicle, the braking of the vehicle, the undue proximity of the vehicle to another vehicle, excessive maneuvering, and/or an unduly high velocity of the vehicle.

7. The system according to claim 1, wherein said sensor means (S₁-S₁₁) comprises a sensor (S₁₁) for sensing whether either of the turn indicators of the vehicle is operating to signal for a turn or a change of lanes.

## Patentansprüche

1. Sicherheitssteuersystem für Fahrzeuge, wobei zum Bedienen eines Telefons oder Telematikgeräts erforderlich ist, dass sich beide Hände am Lenkrad befinden und das System eine Telematik-Einrichtung (10, 11) und Sensoreinrichtung (S₁ - S₁₁) zum Erfassen eines möglicherweise gefährlichen Zustands aufweist, um bei Erfassen eines solchen Zustands die Benutzung der Telematik-Einrichtung (10, 11) automatisch zu deaktivieren,
**dadurch gekennzeichnet, dass** die Sensoreinrichtung (S₁ - S₁₁) zwei Sensoren (S₁, S₂) aufweist, die an einem Lenkrad (4) angebracht ist, um durch die beiden Hände des Fahrers des Fahrzeugs (2) ergriffen zu werden und ein Unterbrechen oder Deaktivieren der Telematik-Einrichtung (10, 11) bewirken, wenn erfasst wird, dass nicht beide Hände des Fahrers das Lenkrad (4) ergreifen, während das Fahrzeug in Beuvegung ist, wobei
die zwei Sensoren (S₁, S₂) elektrische Schalter sind, die so angeordnet, dass sie entweder mit aufwärts oder abwärts gerichteten Daumen der entsprechenden Hand des Fahrers betätigbar sind, wenn das Lenkrad (4) passend umgriffen wird, wodurch dem Fahrer mit seiner/ihrer Hand auf dem betätigten Sicherheitsschalter ermöglicht wird, mit dem Sicherheitsschalter durch Optionen zu klicken, bis eine benötigte Auswahl erreicht ist.

2. System nach Anspruch 1, wobei das System fähig ist, den Signalton eines eingehenden Anrufs abzuschalten.

3. System nach Anspruch 1, wobei die Telematik-Einrichtung (10, 11) ein Telefon ist.

4. System nach Anspruch 1, wobei die zwei Sensoren (S₁, S₂) etwa auf oder zwischen den "zwei" und "zehn" und "drei" bis "neun" - Uhr-Positionen des Lenkrades (4) angeordnet sind.

5. System nach Anspruch 1, wobei das Fahrzeug ferner einen Computer oder ein Multifunktions-Telematik-Gerät mit Internetzugang aufweist, um Faxnachrichten oder e-Mails zu senden und/oder zu empfangen, oder WAN oder Webseiten aufzurufen, und wobei die Sensoren (S₁, S₁₁) den Zugriff deaktivieren, wenn erfasst wird, dass nicht beide Hände des Fahrers das Lenkrad (4) ergreifen, während das Fahrzeug in Beuvegung ist und die riskanten, gefährlichen Bedingungen vorliegen.

6. System nach Anspruch 1, wobei die Sensoreinrichtung (S₁ - S₁₁) Erfassungsmittel für Beschleunigung, Verzögerung, Ein- oder Ausfahren von einer Autobahn, Überholen, Spurwechsel, Gangwechsel, Kupplungsbetätigung, Rückfahrzustand des Fahrzeugs, Bremsbetrieb des Fahrzeugs, unlässige Annäherung des Fahrzeugs an ein anderes Fahrzeug, übermäßige Fahrmanöver und/oder unzulässig hohe Geschwindigkeit des Fahrzeugs aufweist.

7. System nach Anspruch 1, wobei die Sensoreinrichtung (S₁ - S₁₁) einen Sensor (S₁₁) aufweist, um zu erfassen, ob einer der Fahrtrichtungsanzeiger des Fahrzeugs zur Signalisierung des Abbiegens oder eines Fahrbahnwechsels betätigt ist.

## Revendications

1. Système de commande de sécurité pour véhicules (2) nécessitant que les deux mains se trouvent sur le volant de direction afin que le conducteur fasse fonctionner un téléphone ou une télématique, ledit système comprenant un dispositif télématique (10, 11) et des moyens capteurs (S₁-S₁₁) pour détecter une condition potentiellement dangereuse et pour désactiver automatiquement l'utilisation du dispositif télématique (10, 11) lors de la détection d'une telle condition ;
**caractérisé en ce que** lesdits moyens capteurs (S₁-S₁₁) comprennent
deux capteurs (S₁, S₂) montés sur un volant de direction (4) destiné à être saisi par les deux mains du conducteur du véhicule (2) et efficaces pour suspendre ou désactiver le dispositif télématique (10, 11) lorsque les deux mains du conducteur ne sont pas détectées comme saisissant ledit volant de direction (4) alors que le véhicule (2) est en mouvement, dans lequel
les deux capteurs (S₁, S₂) sont des commutateurs électriques qui sont agencés pour être actionnés dans la position pouces vers le haut ou la position pouces vers le bas par la main respective du conducteur lorsqu'il saisit correctement le volant de direction (4), moyennant quoi
le conducteur avec sa main sur le commutateur de sécurité actionné peut faire défiler des options avec le commutateur de sécurité jusqu'à ce qu'il obtienne une sélection qui est nécessaire.

2. Système selon la revendication 1, dans lequel ledit système est capable de désactiver le signal de sonnerie d'un appel entrant.

3. Système selon la revendication 1, dans lequel ledit dispositif télématique (10, 11) est un téléphone.

4. Système selon la revendication 1, dans lequel lesdits deux capteurs (S₁, S₂) sont situés approximativement sur ou entre les positions de « deux » et « dix » heures et les positions de « trois » et neuf » heures du volant de direction (4).

5. Système selon la revendication 1, dans lequel ledit véhicule comprend en outre un ordinateur ou un dispositif télématique multifonctionnel permettant d'accéder à Internet pour transmettre et/ou recevoir des fax ou e-mail, WAN ou naviguer sur des sites Web, et dans lequel lesdits moyens capteurs (S₁, S₁₁) désactivent ledit accès lorsque les deux mains du conducteur ne sont pas détectées comme saisissant le volant de direction (4) alors que le véhicule est en mouvement et les conditions dangereuses risquées sont en cours.

6. Système selon la revendication 1, dans lequel les moyens capteurs (S₁-S₁₁) comprennent des moyens pour détecter l'accélération, la décélération, la jonction à une, ou la sortie d'une, autoroute, le passage, le changement de voies, le changement de vitesses, l'appui sur l'embrayage, une condition de marche arrière du véhicule, le freinage du véhicule, la proximité excessive du véhicule à un autre, les manoeuvres excessives, et/ou une vitesse excessivement élevée du véhicule.

7. Système selon la revendication 1, dans lequel lesdits moyens capteurs (S₁-S₁₁) comprennent un capteur (S₁₁) pour détecter si l'un ou l'autre des clignotants du véhicule fonctionne pour signaler pour un virage ou un changement de voies.
